# EUROPÄISCHE PATENTSCHRIFT

(11) **EP 2 633 883 B1**
(45) Veröffentlichungstag und Bekanntmachung des Hinweises auf die Patenterteilung: **10.12.2014**
(21) Anmeldenummer: 12196257.5
(22) Anmeldetag: 10.12.2012
(51) Int. Cl.: A61N 1/05

(54) **Elektrodenkatheter, insbesondere zur Kardialtherapie**
Electrode catheter, in particular for cardiac therapy
Cathéter à électrodes, notamment pour la thérapie cardiaque

(30) Priorität: 28.02.2012 US 201261603959 P
(43) Veröffentlichungstag der Anmeldung: 04.09.2013
(60) Teilanmeldung: 14166500.0
(73) Patentinhaber: BIOTRONIK SE & Co. KG, 12359 Berlin (DE)
(72) Erfinder: Weitzig, Pierre, 10249 Berlin (DE); Palm, Jochen, 15831 Mahlow (DE); Jadwizak, Detmar, 15537 Erkner (DE); Fründt, Carsten, 13057 Berlin (DE); Hillebrand, Gordon, 12157 Berlin (DE)
(74) Vertreter: Lindner-Vogt, Karin L.

(56) Entgegenhaltungen:
- WO-A1-00/32130
- WO-A1-01/03595
- WO-A1-97/42893
- US-A1- 2003 023 295

## Beschreibung

Die Erfindung betrifft einen Elektrodenkatheter, insbesondere zur Kardialtherapie, der einen langgestreckten, schlauchartigen Katheterkörper, eine Ringelektrode vor dem distalen Ende des Elektrodenkatheters zur Abgabe oder Messung eines elektrischen, insbesondere elektrokardialen Signals über ihre äußere Elektrodenkontaktfläche und eine Zuleitung zum elektrischen Anschluss der Ringelektrode aufweist.

Die vorstehenden Merkmale geben die einfachste Grundkonfiguration eines Elektrodenkatheters mit Ringelektrode wieder, wie sie aus vielen Druckschriften zum Stand der Technik, wie beispielsweise aus der US 6,882,886 oder der US 7,493,173 bekannt sind. In aller Regel weisen solche Elektrodenkatheter, welche auch als Elektrodenleitungen zum dauerhaften Verbleib im Körper bekannt sind, neben der erwähnten Ringelektrode auch eine Spitzenelektrode bei einer bipolaren Ausführung oder weitere Ringelektroden bei einem multipolaren Elektrodenkatheter auf.

Die der Erfindung zugrunde liegende Problematik ergibt sich aus den sich teilweise widersprechenden Anforderungen bei der Platzierung eines Elektrodenkatheters, beispielsweise in einem Herzkranzgefäß. Zum einen soll der Elektrodenkatheter so implantiert werden, dass eine möglichst gute Verankerung des Katheters stattfinden kann. Die so vom Implanteur gefundene Position muss dann allerdings nicht die Optimalposition für die Erzielung möglichst guter Reizschwellenwerte an der Ringelektrode sein. Auch besteht das Problem, dass bei Stimulationskathetern eine unerwünschte Phrenicus-Stimulation (Schluck-auf-Stimulation) einhergehen kann, was bisher an sich nur durch eine Repositionierung der Ringelektrode abgestellt werden kann. Dies wiederum kann zu einer Verschlechterung der Verankerung des Katheters führen.

Ein aus dem Stand der Technik bekannter Lösungsansatz für diese Problematik besteht darin, eine multipolare, beispielsweise vierpolige Elektrodenleitungen vorzusehen, bei der neben der Spitzenelektrode drei in Abstand zueinander angeordnete Ringelektroden vorgesehen sind. Nach der Implantation des Katheters wird dann diejenige Ringelektrode zur Messung elektrokardialer Signale oder zur Abgabe von Reizimpulsen verwendet, die bei der jeweiligen Verankerungsposition die besten Resultate hierfür liefert. Von Nachteil bei dieser Konfiguration ist allerdings die Tatsache, dass sich durch die mehrfachen Ringelektroden die Konstruktion des Katheters erheblich komplexer gestaltet und eine Vielzahl kostenintensiver Bauelemente eingesetzt werden müssen, die in der praktischen Anwendung der Elektrode dann keinerlei Anwendung mehr finden.

Ein weiterer aus dem Stand der Technik bekannter Ansatz besteht darin, die Ringelektrode in Axialrichtung relativ zum Katheterkörper verschiebbar auf diesem zu lagern (siehe WO 00/32130).

Vorteilhafterweise hat bei letzterem Ansatz der Implanteur also die Möglichkeit, nach dem Positionieren und Fixieren des Elektrodenkatheters beispielsweise in einem Herzkranzgefäß die Position der Ringelektrode so zu variieren, bis innerhalb des verfügbaren Verschiebeweges die besten Reizschwellenwerte erzielt werden. Der Elektrodenkatheter als solcher kann dabei an seinem Verankerungsplatz bleiben, sodass eine Verminderung der Fixierungskräfte durch eine Verschiebung und demzufolge drohende Dislokationen des Katheters vermieden werden können. Auch bei einer eventuellen Phrenicus-Stimulation kann der Implanteur die Ringelektrode ohne Lageveränderung des Katheters selbst repositionieren, um die Schluckauf-Reizung zu unterbinden.

Bevorzugtermaßen erfolgt die Verschiebung der Ringelektrode durch eine Antriebsvorrichtung am Elektrodenkatheter von seinem proximalen Ende im implantierten Zustand aus.

Für diese Antriebsvorrichtung können unterschiedlichste konstruktive Konzepte umgesetzt werden. So benutzt eine alternative, der hiesigen Erfindung entsprechende Ausführungsform die ohnehin vorhandene, für den elektrischen Anschluss der Ringelektrode vorgesehene Anschlusswendel, die dann zumindest auf einer Teillänge offen gewickelt ist. "Offen" im Sinne der Patentanmeldung bedeutet, dass sich zwischen dem einzelnen, um 360° umlaufenden Draht eines Wendelabschnittes, der an seinen beiden Endes am Draht des nächsten Wendelabschnittes verbunden ist, ein definierter Raum befindet. Das heißt, die Drähte sind zueinander beabstandet. Nahe der Ringelektrode ist ein sogenannter Steigungsgeber im Bereich der offenen Wicklung angeordnet, der mit der Anschlusswendel im Raum zwischen den Wendelabschnitten derart in Eingriff steht, dass eine Rotation der Anschlusswendel ihre Längenänderung und damit eine Axialverschiebung der Ringelektrode hervorruft. Die Anschlusswendel der Ringelektrode weist bei dieser Konfiguration also eine Doppelfunktion auf. Die Konstruktion der Antriebsvorrichtung insgesamt ist dadurch vergleichsweise einfach und zuverlässig. Im Hinblick auf die großen Längen des Katheterkörpers und der Anschlusswendel stellen die erzielten Längenänderungen der Wendel im Bereich von maximal 40 bis 50 mm in der Praxis wohl eher 20 bis 30 mm, kein Problem dar. Die entstehenden Federkräfte bleiben klein, sodass die Selbsthemmung der Wendel im Steigungsgeber und damit die konstante Beibehaltung der einmal eingestellten Position der Ringelektrode nicht beeinträchtigt werden.

Gemäß einer bevorzugten Weiterbildung der Erfindung passt sich der längenflexibel ausgebildete Außenschlauch des Elektrodenkatheters entsprechend der Axialverschiebung der Ringelektrode in seiner Länge an. Dies ist in der Regel problemlos möglich, da dieser Außenschlauch meist aus Silikonmaterial sehr hoher Elastizität besteht. Ferner ist die absolute Länge des Schlauches von beispielsweise 700 mm sehr viel größer als der Verschiebeweg der Ringelektrode, sodass die prozentuale Längenänderung des Außenschlauches gering bleibt.

Ein Detailproblem bei der konstruktiven Umsetzung der Erfindung ist die Abdichtung des die Außenwendel aufnehmenden Lumens des Außenschlauchs gegen durch die Ringelektrode von ihrer distalen Seite aus eindringende Körperflüssigkeiten.

Eine besonders rationelle Konstruktionsweise für die Abdichtung des Lumens für die Außenwendel erfolgt über einen in die Ringelektrode integrierten Dichtring, der in der nach distal weisenden Öffnung der Ringelektrode zwischen dieser und dem Innenschlauch des Elektrodenkatheters eingesetzt ist. Dieser Innendichtring entspricht in seiner Funktion einem O-Ring, wie er aus dem Maschinenbau bekannt ist. Seine Positionsgenauigkeit und dauerhafte Fixierung kann dabei noch durch eine in die Innenfläche der Ringelektrode eingebrachte Ringnut unterstützt werden, in der der Dichtring sitzt. Bei der vorstehend genannten Innendichtring-Variante kann auf das distal vor der Ringelektrode angeordnete Schlauchstück vollkommen verzichtet werden. Damit ist auch möglichen Verwerfungen beim Verschieben der Ringelektrode vorgebeugt. Gegebenenfalls kann ein System von mehreren Dichtungen in der Ringelektrode in Axialrichtung ineinander - vorzugsweise mit jeweils geringeren Anpresskräften - realisiert werden. Damit können die Reibungskräfte insgesamt verringert werden, um so ein Verschieben der Ringelektrode auf dem Innenschlauch zu erleichtern.

Um eine Verdrillung der den Katheterkörper bildenden, koaxial ineinandersitzenden bzw. in längsaxialer Richtung aufgereihten Komponenten, wie Außenschlauch, Ringelektrode, Innenschlauch und Schlauchstück zwischen Ring- und Spitzenelektrode zu vermeiden, ist gemäß einer weiteren bevorzugten Ausführungsform vorgesehen, dass der Außenschlauch bzw. das Schlauchstück mit der Ringelektrode über darin drehbar, abgedichtet eingreifende Verbindungshülsen verbunden sind. Damit wird eine entsprechende Drehung der Außenwendel und der Ringelektrode nicht auf den Außenschlauch und das Schlauchstück übertragen. Diese werden nur in axialer Richtung gestaucht oder gedehnt, was materialschonender als eine Verdrillung ist.

Um einen sauberen Längsverbund der Ringelektrode mit dem Außenschlauch und dem Schlauchstück zur Spitzenelektrode hin zu gewährleisten, sind die Verbindungshülsen in Längsrichtung formschlüssig mit der Ringelektrode zumindest mittelbar verbunden.

Eine besonders geschickte Weiterbildung bietet sich schließlich für die elektrische Kontaktierung der Ringelektrode über die Außenwendel. Letztere ist mit ihrem ringelektrodenseitigen Ende über eine mit der Ringelektrode in elektrischem Kontakt stehende Kontakthülse verbunden, die vorzugsweise zwischen der Ringelektrode und einer der Verbindungshülsen drehbar gelagert ist.
Reibungsarme Oberflächen auf Innen-, Außenschlauch, Schlauchstück und/oder Faltenbalg können insbesondere durch Gleitbeschichtungen erzeugt werden.

Die mit der Erfindung erzielbaren Vorteile sind nochmals kurz wie folgt zusammcnzufassen:
- Durch die individuell auf dem Elektrodenkatheter verschiebbare Ringelektrode werden bestmögliche Reizschwellenwerte erzielt.
- Bei der Anpassung der Ringelektrodenposition muss nicht der gesamte Elektrodenkatheter bzw. der distale, im Herzkranzgefäß liegende Bereich des Elektrodenkatheters verschoben werden.
- Das Risiko von Dislokation nach der Implantation wird erheblich reduziert, da der Fixiervorgang des Elektrodenkatheters im Herzkranzgefäß vom Vorgang der Positionsoptimierung der Ringelektrode getrennt ist. Es kann also immer eine geeignete Liegeposition für den Katheter mit ausreichenden Haltekräften gefunden werden, bevor die bestmögliche Platzierung für die Ringelektrode eingestellt wird.
- Es werden keine unnötigen, kostenintensiven und im Realbetrieb nicht verwendeten Bauteile verbaut, wie dies beispielsweise bei einer quadropolaren Elektrode der Fall ist.
- Eine eventuell auftretende Phrenicus-Stimulation kann durch ein Repositionieren der Ringelektrode ohne Verschiebung des gesamten Elektrodenkatheters vermieden werden.

Weitere Merkmale, Einzelheiten und Vorteile der Erfindung ergeben sich aus der nachfolgenden Beschreibung, in der Ausführungsbeispiele anhand der beigefügten Zeichnungen näher erläutert werden. Es zeigen:
- Fig. 1: den distalen Endbereich eines Elektrodenkatheters mit Spitzen- und Ringelektrode in perspektivischer Ansicht,
- Fig. 2: eine ausschnittsweise Vergrößerung der Abbildung gemäß Fig. 1 bei weggelassenem Außenschlauch im Bereich der Ringelektrode mit Außenwendel,
- Fig. 3 und 4: perspektivische Teilansichten des distalen Endbereiches eines Elektrodenkatheters in zwei weiteren Ausführungsformen,
- Fig. 5: eine Ansicht eines Elektrodenkatheters analog Fig. 3 und 4 in weiteren Ausführungsformen,
- Fig. 6: eine ausschnittsweise, teilweise weggebrochene Ansicht des Elektrodenkatheters gemäß Fig. 5 im Bereich des Faltenbalges,
- Fig. 7: eine perspektivische Ansicht des distalen Endes eines Elektrodenkatheters in einer weiteren Ausführungsform,
- Fig. 8: eine perspektivische, ausschnittsweise Ansicht des Elektrodenkatheters gemäß Fig. 7,
- Fig. 9: eine perspektivische, teilweise weggebrochene Ansicht eines Elektrodenkatheters mit einer Anbindung der Ringelektrode in einer weiteren alternativen Ausführungsform, und
- Fig. 10: eine perspektivische, teilweise weggebrochene Teildarstellung der Anbindung des distalen Schlauchstücks an die Ringelektrode aus Fig.9.

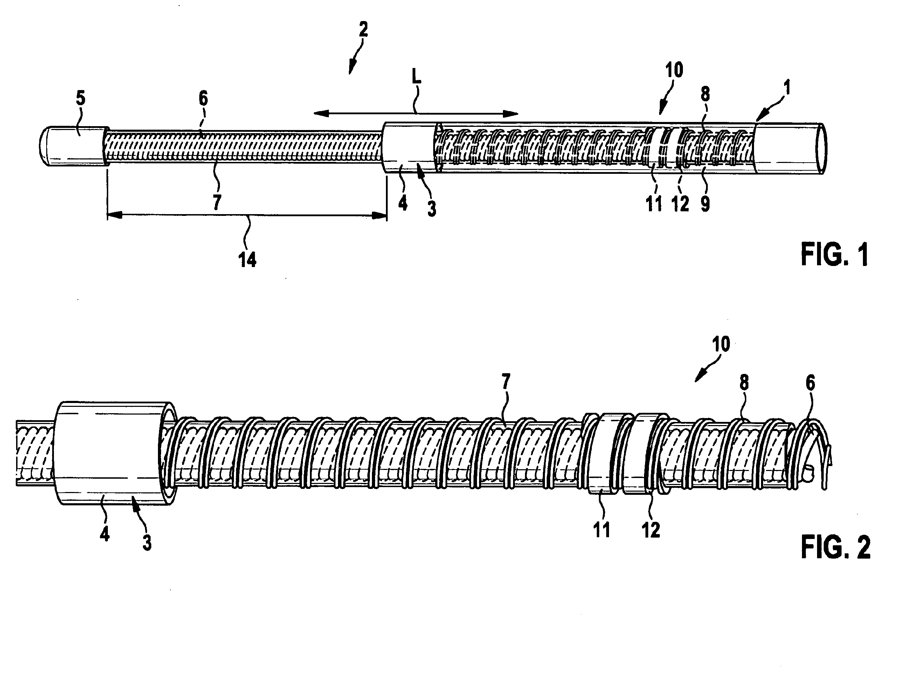

Anhand von Fig. 1 ist der grundsätzliche Aufbau eines bipolaren Elektrodenkatheters zu erörtern, wie er für alle nachfolgend erläuterten und dargestellten Ausführungsformen der Erfindung zutrifft. So weist der Elektrodenkatheter einen langgestreckten, schlauchartigen Katheterkörper 1 auf, der an seinem (nicht dargestellten) proximalen Ende mit entsprechenden Manipulationseinrichtungen zum Handhaben des Katheters und elektrischen Anschlüssen für die Kontaktierung versehen ist. Im Bereich des distalen Endes 2 des Katheterkörpers ist dieser mit einer vor dem distalen Ende 2 angeordneten Ringelektrode 3 versehen, die zur Messung eines elektrokardialen Signals oder zur Abgabe eines entsprechenden Reizsignals über ihre äußere Elektrodenkontaktfläche 4 dienen kann. Am distalen Ende 2 ist ferner eine Spitzenelektrode 5 angeordnet, die den Katheterkörper 1 in Form einer Kappe oder eines Bechers distal abschließt.

Zum elektrischen Anschluss der Spitzenelektrode ist eine Innenwendel 6 vorgesehen, die in einem Innenschlauch 7 durch den gesamten Katheterkörper 1 hindurch bis zur Spitzenelektrode 5 verläuft und dort durch übliche Fügeverfahren, wie Crimpen, Schweißen oder Löten festgelegt ist.

Auf dem Innenschlauch 7 sitzt eine Außenwendel 8, die bis zur Ringelektrode 3 verläuft und zu deren elektrischer Kontaktierung daran angeschlossen ist. Nach außen ist der Katheterkörper 1 im Bereich der Außenwendel 8 durch einen Außenschlauch 9 aus flexiblem, sehr dehnfähigen Silikonmaterial abgeschlossen. Insgesamt liegen also Innenwendel 6, Innenschlauch 7, Außenwendel 8 und Außenschlauch 9 koaxial ineinander.

Allen Ausführungsformen ist gemeinsam, dass die Ringelektrode 3 in längsaxialer Richtung L entlang des Katheterkörpers 1 über eine bestimmte Distanz von beispielsweise 20 bis 50 mm verschiebbar ist. Die Verschiebbarkeit wird dabei durch eine als Ganzes mit 10 rein funktional bezeichnete Antriebsvorrichtung hervorgerufen, die am nicht gezeigten proximalen Ende des Katheterkörpers 1 betätigbar ist.

Bei der in Fig. 1 und 2 gezeigten Ausführungsform eines Elektrodenkatheters wird diese Antriebsvorrichtung 10 durch die zumindest im Bereich proximal vor der Ringelektrode 3 offen gewickelte Außenwendel 8 in Kooperation mit einem auf dem Innenschlauch 7 ortsfest sitzenden Steigungsgeber 11 realisiert. Bei diesem Steigungsgeber 11 handelt es sich um eine Hülse, die eine schraubenlinienförmige Nut 12 entsprechend der Wicklungssteigung der Außenwendel 8 aufweist. Wie in Fig. 2 deutlich erkennbar ist, verläuft die Außenwendel 8 über ca. zwei Windungen in dieser Nut 12 des Steigungsgebers 11. Wird die Außenwendel 8 nun um ihre Längsachse in Rotation versetzt, wird eine Verschiebung der Außenwendel 8 in längsaxialer Richtung L hervorgerufen, die sich auf die Ringelektrode 3 überträgt. Im gezeigten Ausführungsfall ist die Außenwendel 8 dazu stoffschlüssig mit der Ringelektrode, beispielsweise durch Löten oder Schweißen verbunden, sodass neben der mechanischen Verbindung auch eine elektrische Kontaktierung der Ringelektrode 3 erfolgt.

Am nicht gezeigten proximalen Ende ist die Außenwendel 8 mit einem drehbaren Steckerelement, das vom Implanteur gegriffen und in Rotation versetzt werden kann, versehen. Damit lässt sich die Drehbewegung der Außenwendel 8 manuell erzeugen.

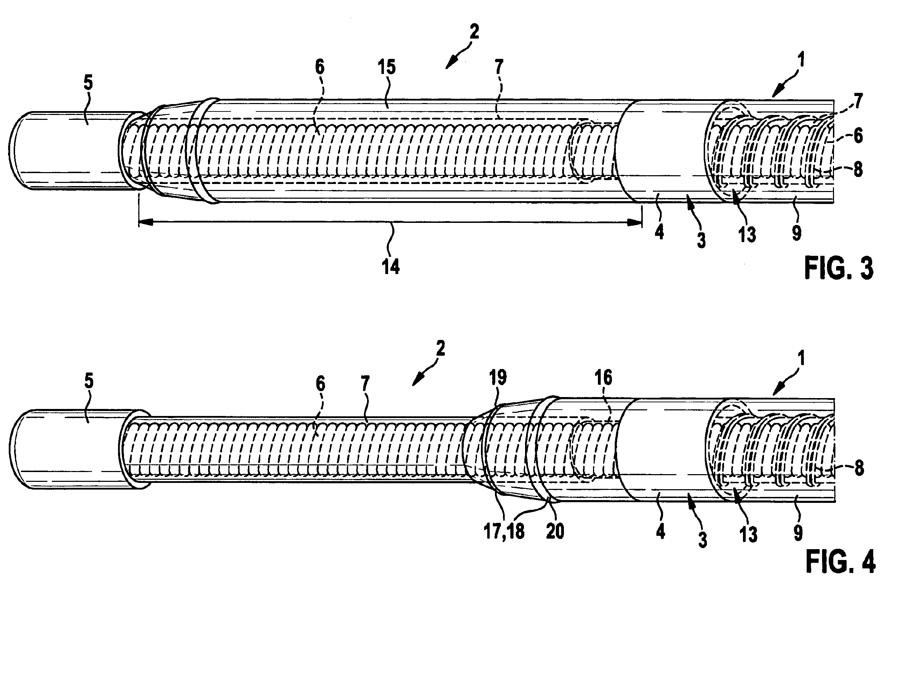

Die verschiedenen Ausführungsformen gemäß den Fig. 3 bis 8 greifen bezüglich der Verschiebung der Ringelektrode 3 auf die anhand von Fig. 1 und 2 erläuterte Konstruktion zurück. Insoweit ist eine nochmalige Erläuterung in diesem Zusammenhang entbehrlich.

Die verschiedenen Ausführungsformen gemäß der Fig. 3 bis 8 sind auf die Detailproblematik der Abdichtung des Katheterkörpers 1 im Bereich des distalen Endes 2 und insbesondere des Außenlumens 13 gerichtet, in dem die Außenwendel 8 zur Ringelektrode 3 geführt ist.

Bei der in Fig. 3 gezeigten Konfiguration ist der Abstand 14 zwischen Ringelektrode 3 und Spitzenelektrode 5 durch ein Schlauchstück 15 abgedeckt, das koaxial auf dem Innenschlauch 7 sitzt und in seinem unbelasteten Zustand im Außendurchmesser der Ring- und Spitzenelektrode 3, 5 entspricht. Das Material dieses Schlauchstückes 15 ist ein Silikon geringer Härte von beispielsweise 50 shore. Die zur Spitzenelektrode 5 hin gewandte Stimkante des Schlauchstücks 15 ist umlaufend mit dem Innenschlauch 7 verklebt, womit der Innenraum abgedichtet ist.

Bei der Verschiebung der Ringelektrode 3 in Richtung zur Spitzenelektrode 5 hin kann eine Aufstauchung des Schlauchstücks 15 erfolgen. Die damit verbundene Durchmesservergrößerung kann sich vorteilhaft auf die Fixierung des Katheters in einem Herzgefäß auswirken. Aufgrund der geringen Härte des Schlauchsstücks 15 sind allerdings alle Dehn- und Stauchprozesse ohne erheblichen Kraftaufwand möglich, wodurch die Klebestellen des Schlauchstücks 15 zu den benachbarten Elementen nicht über Gebühr strapaziert werden. Auch ist die Länge des Schlauchstückes 15 ohnehin so groß, dass die Dehnvorgänge denen gegenüber nicht ins Gewicht fallen.

Bei der in Fig. 4 gezeigten Ausführungsform ist das Lumen der Außenwendel 8 mit dem Innenraum der Ringelektrode 3 durch ein kurzes Dichtschlauchstück 16 abgedichtet, das mit seinem proximalen Ende an die Ringelektrode 3 dicht angeklebt ist. Zum seinem distalen Ende hin läuft das Dichtschlauchstück 16 konisch aus. Die Abdichtung auf dem die Innenwendel 6 zur Spitzenelektrode 5 hin umgebenden Innenschlauch 7 erfolgt durch die Eigenspannung des Dichtschlauchstücks 16, wird aber durch Beaufschlagungsringe 17, 18 in Form von Schrumpfringen verstärkt, die in Außennuten 19, 20 des Dichtschlauchstücks 16 eingesetzt sind und letzteres radial nach innen auf den Innenschlauch 7 pressen. Dadurch wird bei einer Drehung der Ringelektrode 3 über die Außenwendel 8 mit entsprechender Längsverschiebung der Ringelektrode 3 kein nennenswertes Drehmoment auf den Innenschlauch 7 übertragen. Vielmehr gleitet das Dichtschlauchstück 16 auf dem Innenschlauch 7.

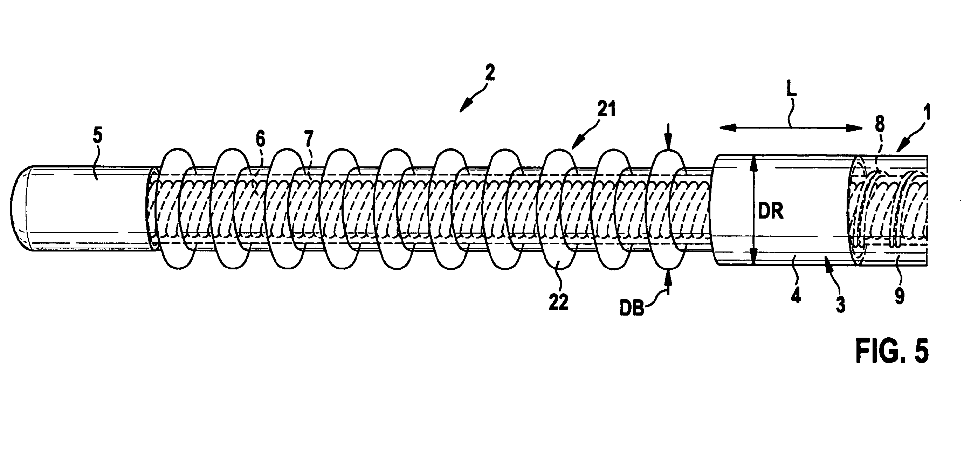

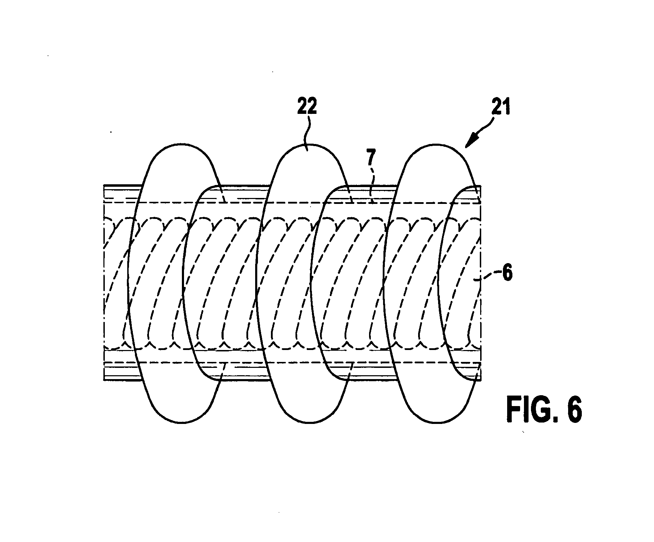

In den Fig. 5 und 6 ist eine Alternative für das Schlauchstück 15 gemäß Fig. 3 gezeigt. Es handelt sich dabei um einen Faltenbalg 21 aus Silikonmaterial, der mit seinem zur Spitzenelektrode 5 hin gewandten Ende wiederum am Innenschlauch 7 durch eine Rundum-Verklebung festgelegt und hermetisch dicht angeschlossen ist. Aufgrund des sehr flexiblen Materials des Faltenbalgs 21 und der grundsätzlich guten Dehnfähigkeit einer Balgkonfiguration kann sich der Faltenbalg 21 mit denkbar geringem Kraftaufwand in seiner Länge an die Positionsänderungen der Ringelektrode 3 anpassen. Der Außendurchmesser DB der Balgfalten entspricht im Wesentlichen dem Außendurchmesser DR der Ringelektrode, was einer problemlosen Einführbarkeit des Elektrodenkatheters in den Patientenkörpers dienlich ist. Die Balgfalten 22 unterstützen ferner aufgrund ihrer Formgebung die Fixierung des Katheterkörpers 1 mit seinem distalen Ende 2 in einem Herzgefäß des Patienten.

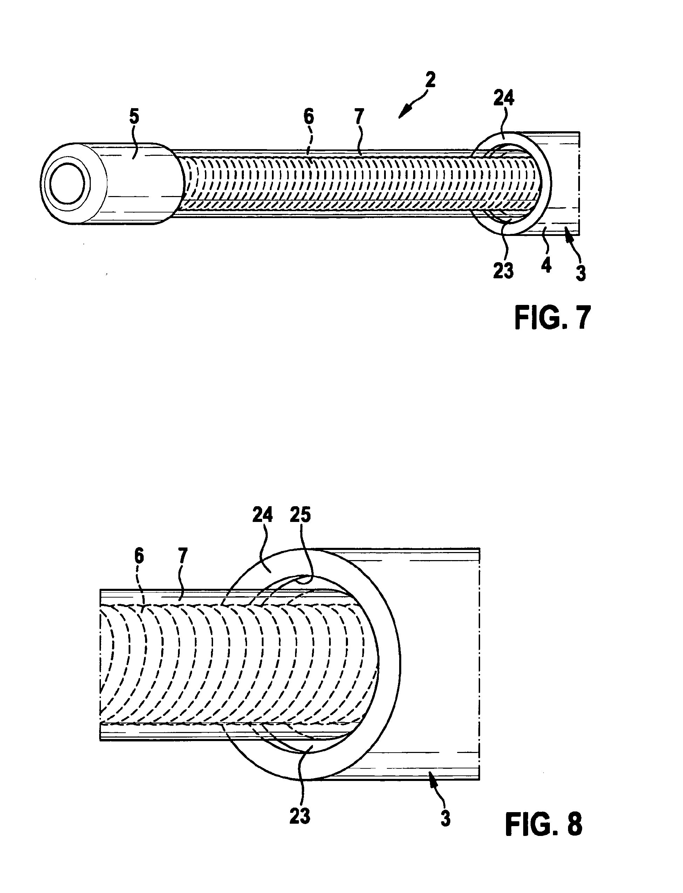

Eine weitere Ausführungsform des Katheterkörpers 1 im Bereich seines distalen Endes 2 ist in den Fig. 7 und 8 gezeigt. Hier läuft die Innenwendel 6 mit ihrem Innenschlauch 7 ohne weitere Abdeckung bis zur Spitzenelektrode 5 durch. Der Innenraum der Ringelektrode 3 und des sich in proximaler Richtung anschließenden Außenschlauchs 9 wird durch einen oder mehrere Innendichtringe 23 in der Ringelektrode 3 abgedichtet. Der Innendichtring 23 ist dabei in eine umlaufende Innennut 25 vor der distal weisenden Stirnkante 24 in der Ringelektrode zuverlässig fixiert. Der Innendichtring 23 sitzt dicht auf dem Innenschlauch 7, sodass der hinter ihm liegende Lumenbereich abgedichtet ist. Sofern mehrere Innendichtringe hintereinander positioniert werden, können die einzelnen Ringe weniger stark den Innenschlauch 7 beaufschlagen, sodass die Reibungskräfte verringert und ein Verschieben der Ringelektrode 3 erleichtert wird.

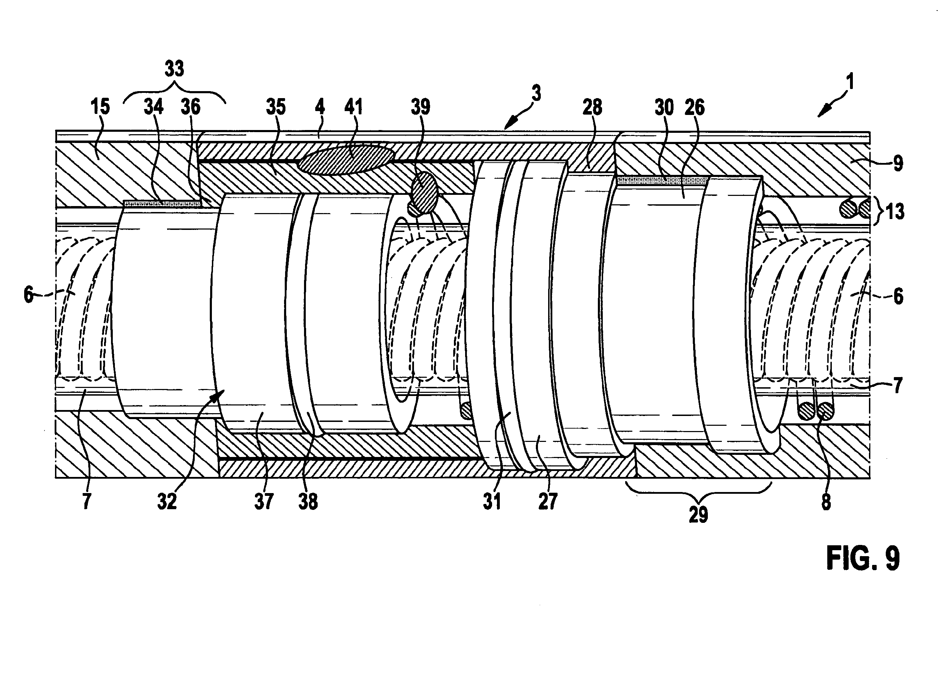

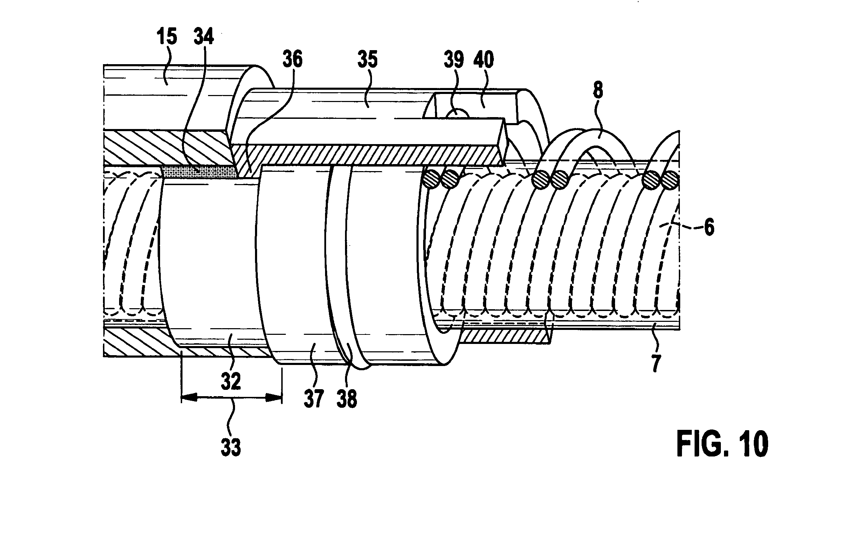

Die in den Fig. 9 und 10 dargestellte Ausführungsform eines Elektrodenkatheters löst das Problem, dass durch die Drehung der Außenwendel 8 zur längsaxialen Verschiebung der Ringelektrode 3 mithilfe des Steigungsgebers 11 und der notwendigen Abdichtung der Lumen des Elektrodenkatheters einerseits flüssigkeitsdichte Übergänge zwischen benachbarten Komponenten vorhanden sein müssen, andererseits jedoch bei entsprechenden Klebungen die Drehbewegungen von Außenwendel 8 und Ringelektrode 3 auf die benachbarten Schläuche, wie den Außenschlauch 9 und insbesondere das Schlauchstück 15 übertragen werden würden. Die damit verbundenen Verdrillungen dieser Komponenten sind auf Dauer materialermüdend und sollten vermieden werden.

Zur Lösung dieser Problematik ist bei der in Fig. 9 und 10 gezeigten Ausführungsform nun zum einen vorgesehen, auf der proximalen Seite der Ringelektrode 3 den Außenschlauch 9 über eine erste Verbindungshülse 26 mit der Ringelektrode 3 zu verbinden. Diese Verbindungshülse 26 wird von der distalen Seite her bei der Montage in die Ringelektrode 3 eingeschoben und bildet mit einer distalen Ringschulter 27 einen Anschlag für einen proximalen Ringvorsprung 28 am proximalen Ende der Ringelektrode 3. Die Verbindungshülse 26 ist somit drehbar in der Ringelektrode 3, in längsaxialer Richtung jedoch formschlüssig in der Ringelektrode 3 gehalten und kann nicht in Proximalrichtung ausgezogen werden.

Der Außenschlauch 9 ist mit dem aus der Ringelektrode 3 in proximaler Richtung herausstehenden Zylinderabschnitt 29 der Verbindungshülse 26 über eine Verklebung 30 hermetisch dicht verbunden, was zum einen zur Abdichtung des Außenlumens 13 beiträgt. Zum anderen ist im Bereich der Ringschulter 27 ein Dichtungsring 31 zwischen der Verbindungshülse 26 und der Ringelektrode 30 vorgesehen, sodass der Innenraum komplett von der proximalen Seite her abgedichtet ist.

Zum Schlauchstück 15 hin ist ebenfalls eine Verbindungshülse 32 vorgesehen, deren distales Ende aus der Ringelektrode 3 in distaler Richtung hervorsteht. Auf diesem entsprechenden Zylinderabschnitt 33 ist das proximale Ende des Schlauchstückes 15 hermetisch dicht durch eine Verklebung 34 befestigt.

Zwischen dem in der Ringelektrode 3 befindlichen Teil der Verbindungshülse 32 und der Ringelektrode 3 ist eine Kontakthülse 35 eingesetzt, die mit einem nach innen weisenden Ringvorsprung 36 eine entsprechende Ringschulter 37 der Verbindungshülse 32 von der distalen Seite her übergreift. Damit sind analog der Ringelektrode 3 und der Verbindungshülse 26 auch die Verbindungshülse 32 und die Kontakthülse 35 formschlüssig in längsaxialer Richtung gekoppelt. Zwischen der Verbindungshülse 32 und der Kontakthülse 35 ist ein weiterer Dichtungsring 38 eingesetzt, der den Innenraum der Ringelektrode 3 von der distalen Seite her gegen die Kontakthülse 35 abdichtet.

Wie insbesondere aus Fig. 10 deutlich wird, ist die Kontakthülse 35 mit dem distalen Ende der Außenwendel 8 mechanisch und elektrisch über eine entsprechende Verschweißung 39 in einer Kontaktaussparung 40 verbunden. Gleichzeitig ist die Kontakthülse 35 über eine weitere Verschweißung 41 mechanisch und elektrisch mit der Ringelektrode 3 verbunden.

Aufgrund der vorstehend erläuterten Konstruktion der Anbindung der Ringelektrode wird bei einer Drehung der Außenwendel 8 die Kontakthülse 35 zusammen mit der Ringelektrode 3 in eine Drehbewegung versetzt. Gleichzeitig erfolgt über den Steigungsgeber 11 die längsaxiale Verlagerung der Außenwendel 8 und damit der Kontakthülse 35 und der Ringelektrode 3 zur Positionsanpassung letzterer. Die Drehbewegung der Komponenten wird jedoch aufgrund der drehbaren Lagerung der Verbindungshülsen 26, 32 nicht auf diese und die damit verklebten Komponenten, nämlich Außenschlauch 9 und Schlauchstück 15, übertragen, sodass die keinem Drehmoment und damit keiner Verdrillung unterworfen werden. Über die entsprechenden Dichtungsringe 31, 38 sind die inneren Bereiche des Katheterkörpers gleichwohl gegen ein Eindringen von Flüssigkeiten von außen geschützt.

## Patentansprüche

1. Elektrodenkatheter, insbesondere zur Kardialtherapie, umfassend
- einen langgestreckten, schlauchartigen Katheterkörper (1),
- eine Ringelektrode (3) vor dem distalen Ende (2) des Elektrodenkatheters zur Abgabe oder Messung eines elektrischen, insbesondere elektrokardialen Signals über ihre äußere Elektrodenkontaktfläche (4), wobei die Ringelektrode (3) in Längsaxialrichtung (L) relativ zum Katheterkörper (1) verschiebbar auf diesem gelagert ist
- eine Zuleitung (8) zum elektrischen Anschluss der Ringelektrode (3), und
- eine Antriebsvorrichtung (10), mittels derer die Ringelektrode (3) vom proximalen Ende des Elektrodenkatheters aus insbesondere in seinem implantierten Zustand verschiebbar ist,
**dadurch gekennzeichnet, dass**
die Antriebsvorrichtung (10) durch eine zumindest auf einer Teillänge offen gewickelte Anschlusswendel (8) der Ringelektrode (3) gebildet ist, welche einen für den elektrischen Anschluss der Ringelektrode vorgesehenen, gewickelten Draht umfasst, bei dem die Wicklungen in der Längsaxialrichtung zueinander beabstandet sind, wobei ein vorzugsweise nahe vor der Ringelektrode (3) angeordneter Steigungsgeber (11) im Bereich der offenen Wicklung mit der Anschlusswendel (8) derart in Eingriff steht, dass eine Rotation der Anschlusswendel (8) ihre Längenänderung und damit eine Axialverschiebung der Ringelektrode (3) hervorruft.

2. Elektrodenkatheter nach Anspruch 1, welcher zusätzlich einen Innen- sowie Außenschlauch (7, 9), zwischen denen die Anschlusswendel (8) der Ringelektrode (3) verläuft, umfasst, **dadurch gekennzeichnet, dass** sich der längenflexibel ausgebildete Außenschlauch (9) entsprechend der Axialverschiebung der Ringelektrode (3) in seiner Länge anpasst.

3. Elektrodenkatheter nach Anspruch 2, **gekennzeichnet durch** mindestens einen in der nach distal weisenden Öffnung der Ringelektrode (3) zwischen dieser und dem Innenschlauch (7) des Katheterkörpers (1) eingesetzten Innendichtring (23), der das Lumen (13) für die Anschlusswendel (8) abdichtet.

4. Elektrodenkatheter nach Anspruch 3, **dadurch gekennzeichnet, dass** jeder Dichtring (23) in einer in der Innenfläche der Ringelektrode (3) eingebrachte Ringnut (25) sitzt.

5. Elektrodenkatheter nach Anspruch 3 oder 4, **dadurch gekennzeichnet, dass** der Außenschlauch (9) mit der Ringelektrode (3) über darin drehbar, abgedichtet eingreifende Verbindungshülsen (26) verbunden sind.

6. Elektrodenkatheter nach Anspruch 5, **dadurch gekennzeichnet, dass** die Verbindungshülsen (26) in Längsaxialrichtung (L) formschlüssig mit der Ringelektrode (3) zumindest mittelbar verbunden sind.

7. Elektrodenkatheter nach Anspruch 5 oder 6, **dadurch gekennzeichnet, dass** die Anschlusswendel (8) mit ihrem ringelektrodenseitigen Ende über eine mit der Ringelektrode (3) in elektrischem Kontakt stehende Kontakthülse (35) kontaktiert ist, die vorzugsweise zwischen der Ringelektrode (3) und einer der Verbindungshülsen (26) drehbar gelagert ist.

## Claims

1. An electrode catheter, in particular for cardiac therapy, comprising
- an elongated, tube-type catheter body (1),
- a ring electrode (3) before the distal end (2) of the electrode catheter for delivery or measurement of an electrical, in particular electrocardio signal by way of the outer electrode contact surface (4) thereof, wherein the ring electrode (3) is mounted on the catheter body (1) such that it can be displaced relative thereto in the longitudinal axial direction (L)
- a supply lead (8) for the electrical connection of the ring electrode (3), and
- a drive device (10), by means of which the ring electrode (3) can be displaced from the proximal end of the electrode catheter into the implanted state thereof in particular
**characterized in that**
the drive device (10) is formed by a connection coil (8) of the ring electrode (3), which is wound openly at least on a partial length and comprises a wound wire provided for the electrical connection of the ring electrode and in which the windings are spaced apart in the longitudinal axial direction, wherein an advancing element (11) located preferably close before the ring electrode (3) is engaged with the connection coil (8) in the region of the open winding such that rotation of the connection coil (8) induces a change in the length thereof and, therefore, axial displacement of the ring electrode (3).

2. The electrode catheter according to claim 1, which additionaly comprises an inner and an outer tube (7, 9), between which the connection coil (8) of the ring electrode (3) extends, **characterized in that** the outer tube (9), which has a flexible length, adapts its length in accordance with the axial displacement of the ring electrode (3).

3. The electrode catheter according to claim 2, **characterized by** at least one inner sealing ring (23), which is inserted in the distally facing opening of the ring electrode (3) between this and the inner tube (7) of the catheter body (1), which seals the lumen (13) for the connection coil (8).

4. The electrode catheter according to claim 3, **characterized in that** each sealing ring (23) is seated in an annular groove (25) formed in the inner surface of the ring electrode (3).

5. The electrode catheter according to claim 3 or 4, **characterized in that** the outer tube (9) is connected to the ring electrode (3) via connecting sleeves (26), which can rotate therein and engage in a sealing manner.

6. The electrode catheter according to claim 5, **characterized in that** the connecting sleeves (26) are connected at least indirectly in the longitudinal axial direction (L) in a form-fit manner to the ring electrode (3).

7. The electrode catheter according to claim 5 or 6, **characterized in that** the connection coil (8) is contacted to the ring electrode-side end thereof by way of a contact sleeve (35) having electrical contact to the ring electrode (3), which is preferably rotatably disposed between the ring electrode (3) and one of the connecting sleeves (26).

## Revendications

1. Cathéter à électrodes, en particulier pour la thérapie cardiaque, comprenant
- un corps de cathéter (1) tubulaire allongé,
- une électrode annulaire (3) en amont de l'extrémité distale (2) du cathéter à électrodes, destinée à délivrer ou à mesurer un signal électrique, en particulier électro-cardiaque, par sa surface de contact d'électrode extérieure (4), l'électrode annulaire (3) étant montée de façon coulissante sur le corps de cathéter (1), dans la direction axiale longitudinale (L) de celui-ci,
- une ligne d'alimentation (8) pour le raccordement électrique de l'électrode annulaire (3), et
- un dispositif d'entraînement (10) permettant de déplacer l'électrode annulaire (3) à partir de l'extrémité proximale du cathéter à électrodes, en particulier dans son état implanté,
**caractérisé en ce que**
le dispositif d'entraînement (10) est constitué d'au moins un boudin de raccordement (8) de l'électrode annulaire (3), enroulé de façon ouverte au moins sur une partie de la longueur, comprenant un fil enroulé destiné au raccordement électrique de l'électrode annulaire, les enroulements étant espacés les uns des autres dans la direction axiale longitudinale, sachant qu'un indicateur de pente (11) agencé de préférence à proximité de l'électrode annulaire (3) est en prise avec le boudin de raccordement (8) dans la région de l'enroulement ouvert, de telle façon qu'une rotation du boudin de raccordement (8) entraîne une modification de sa longueur et donc un déplacement axial de l'électrode annulaire (3).

2. Cathéter à électrodes selon la revendication 1, comprenant en outre un tube intérieur et un tube extérieur (7, 9), entre lesquels s'étend le boudin de raccordement (8) de l'électrode annulaire (3), **caractérisé en ce que** le tube extérieur (9) conçu élastique en longueur présente une longueur variable en fonction du déplacement axial de l'électrode annulaire (3).

3. Cathéter à électrodes selon la revendication 2, **caractérisé par** au moins une bague d'étanchéité intérieure (23) insérée dans l'ouverture de l'électrode annulaire (3) qui est dirigée vers le côté distal, entre celle-ci et le tube intérieur (7) du corps de cathéter (1), laquelle assure l'étanchéité du lumen (13) pour le boudin de raccordement (8).

4. Cathéter à électrodes selon la revendication 3, **caractérisé en ce que** chaque bague d'étanchéité (23) est logée dans une rainure annulaire (25) située dans la surface intérieure de l'électrode annulaire (3).

5. Cathéter à électrodes selon la revendication 3 ou 4, **caractérisé en ce que** le tube extérieur (9) est relié à l'électrode annulaire (3) par le biais de douilles de raccordement (26) s'engageant dans celui-ci de façon étanche et rotative.

6. Cathéter à électrodes selon la revendication 5, **caractérisé en ce que** les douilles de raccordement (26) sont reliées au moins indirectement par complémentarité de forme à l'électrode annulaire (3) dans la direction axiale longitudinale (L).

7. Cathéter à électrodes selon la revendication 5 ou 6, **caractérisé en ce que** le boudin de raccordement (8) est mis en contact par son extrémité située du côté de l'électrode annulaire, par le biais d'une douille de contact (35) en contact électrique avec l'électrode annulaire (3), laquelle est montée de préférence entre l'électrode annulaire (3) et l'une des douilles de raccordement (26).
